# EUROPEAN PATENT APPLICATION

(11) **EP 3 020 481 A1**
(43) Date of publication of application: **18.05.2016**
(21) Application number: 15160829.6
(22) Date of filing: 25.03.2015
(51) Int. Cl.: B01L 3/00

(54) **MECHANICAL SEPARATOR FOR A BIOLOGICAL FLUID**

(30) Priority: 13.11.2014 US 201462079230 P; 24.02.2015 US 201514629584
(71) Applicant: Becton Dickinson and Company, Franklin Lakes, NJ 07417-1880 (US)
(72) Inventor: Losada, Robert J., New York, 11105 Astoria (US)
(74) Representative: von Kreisler Selting Werner - Partnerschaft von Patentanwälten und Rechtsanwälten mbB

(57) **Abstract**

A mechanical separator and separation assembly for separating a fluid sample into first and second parts within a collection container is disclosed. The mechanical separator has a body having a through-hole for allowing fluid to pass therethrough and includes a first portion, having a first density, and a second portion, having a second density different from the first density. The body defines a longitudinal axis extending perpendicular to the through-hole, and exhibits a first compression value when a force is applied to the body along this axis. The body also defines an axis extending perpendicular to the longitudinal axis and along the through-hole and exhibits a second compression value when a force is applied along this axis. The first compression value is different than the second compression value and may be less than the second compression value.

## Description

### CROSS-REFERENCE TO RELATED APPLICATION

The present application claims priority to United States Provisional Application Serial No. 62/079,230, entitled "Mechanical Separator for a Biological Fluid", filed November 13, 2014, the entire disclosure of which is hereby incorporated by reference.

### BACKGROUND OF THE INVENTION

### Field of the Invention

The subject invention relates to a device for separating higher and lower density fractions of a fluid sample. More particularly, this invention relates to a device for collecting and transporting fluid samples whereby the device and fluid sample are subjected to centrifugation in order to cause separation of the higher density fraction from the lower density fraction of the fluid sample.

### Description of Related Art

Diagnostic tests may require separation of a patient's whole blood sample into components, such as serum or plasma (the lower density phase components), and red blood cells (the higher density phase components). Samples of whole blood are typically collected by venipuncture through a cannula or needle attached to a syringe or an evacuated blood collection tube. After collection, separation of the blood into serum or plasma and red blood cells is accomplished by rotation of the syringe or tube in a centrifuge. In order to maintain the separation, a barrier must be positioned between the higher density and lower density phase components. This allows the separated components to be subsequently examined.

A variety of separation barriers have been used in collection devices to divide the area between the higher density and lower density phases of a fluid sample. The most widely used devices include thixotropic gel materials, such as polyester gels. However, current polyester gel serum separation tubes require special manufacturing equipment to both prepare the gel and fill the tubes. Moreover, the shelf-life of the gel-based separator product is limited. Over time, globules may be released from the gel mass and enter one or both of the separated phase components. Furthermore, commercially available gel barriers may react chemically with the analytes. Accordingly, if certain drugs are present in the blood sample when it is taken, an adverse chemical reaction with the gel interface can occur. Furthermore, if an instrument probe is inserted too deeply into a collection container, then the instrument probe may become clogged if it contacts the gel.

Certain mechanical separators have also been proposed in which a mechanical barrier can be employed between the higher and lower density phases of the fluid sample. Conventional mechanical barriers are positioned between higher and lower density phase components utilizing elevated gravitational forces applied during centrifugation. For proper orientation with respect to plasma and serum specimens, conventional mechanical separators are typically positioned above the collected whole blood specimen prior to centrifugation. This typically requires that the mechanical separator be affixed to the underside of the tube closure in such a manner that blood fill occurs through or around the device when engaged with a blood collection set or phlebotomy needle. This attachment is required to prevent the premature movement of the separator during shipment, handling, and blood draw. Conventional mechanical separators are typically affixed to the tube closure by a mechanical interlock between the bellows component and the closure.

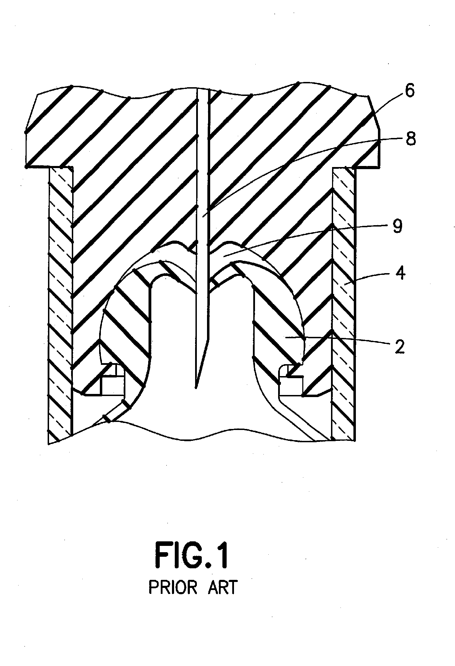

Conventional mechanical separators have some significant drawbacks. As shown in **FIG. 1****,** conventional separators include a bellows **2** for providing a seal with the tube or syringe wall **4.** Typically, at least a portion of the bellows **2** is housed within, or in contact with a closure **6.** As shown in **FIG. 1****,** as the needle **8** enters through the closure **6,** the bellows **2** is depressed. This creates a void **9** in which blood may pool during insertion or removal of the needle. This can result in sample pooling under the closure, device pre-launch in which the mechanical separator prematurely releases during blood collection, trapping of a significant quantity of fluid phases, such as serum and plasma, poor sample quality, and/or barrier failure under certain circumstances. Furthermore, previous mechanical separators are costly and complicated to manufacture due to the complicated multi-part fabrication techniques.

Accordingly, a need exists for a separator device that is compatible with standard sampling equipment and reduces or eliminates the aforementioned problems of conventional separators. A need also exists for a separator device that is easily used to separate a blood sample, minimizes cross-contamination of the higher and lower density phases of the sample during centrifugation, is independent of temperature during storage and shipping, and is stable to radiation sterilization. A need further exists for a unitary separation device that requires fewer relative moving parts and that allows for enhanced ease of introducing a specimen into a collection container.

### SUMMARY OF THE INVENTION

In accordance with an aspect of the present invention, a device for separating a fluid into first and second parts within a container includes a body having a through-hole defined therethrough. The body includes a first portion defining an upper surface of the body, and a second portion defining a lower surface of the body, wherein the first portion and the second portion are interfaced. The body defines a longitudinal axis extending perpendicular to the through-hole, and the body exhibits a first compression value when a force is applied to the body along the longitudinal axis. The body also defines a perpendicular axis extending perpendicular to the longitudinal axis and along the through-hole, and the body exhibits a second compression value when a force is applied to the body along the perpendicular axis. The first compression value is different than the second compression value.

In certain configurations, the first compression value is greater than the second compression value. In certain configurations, the force is exerted to the body during applied rotational force.

In accordance with another aspect of the present invention, a separation assembly for enabling separation of a fluid into first and second phases includes a collection container having a first end, a second end, and a sidewall extending therebetween defining an interior. The separation assembly also includes a separator body having a through-hole defined therethrough. The body includes a first portion defining an upper surface of the body, and a second portion defining a lower surface of the body, wherein the first portion and the second portion are interfaced. The separator body defines a longitudinal axis extending between the upper surface and the lower surface, wherein the separator body exhibits a first compression value when a force is applied to the separator body along the longitudinal axis. The separator body also defines a perpendicular axis extending perpendicular to the longitudinal axis, wherein the separator body exhibits a second compression value when a force is applied to the body along the perpendicular axis. The first compression value is different than the second compression value.

In certain configurations, the first compression value is greater than the second compression value. In certain configurations, the force is exerted to the body during rotational force applied to the container.

### BRIEF DESCRIPTION OF THE DRAWINGS

**FIG. 1** is a partial cross-sectional side view of a conventional mechanical separator.
**FIG. 2** is a perspective view of a mechanical separator in accordance with an embodiment of the present invention.
**FIG. 3** is a front view of the mechanical separator of **FIG. 2****.**
**FIG. 4** is a cross-sectional view of the mechanical separator of **FIG. 2** taken along the longitudinal axis **L** of the mechanical separator as shown in **FIG. 3****.**
**FIG. 5** is a top view of the mechanical separator of **FIG. 2****.**
**FIG. 6** is a side view of the mechanical separator of **FIG. 2****.**
**FIG. 7** is a cross-sectional view of the mechanical separator of **FIG. 2** taken along the longitudinal axis **L** of the mechanical separator as shown in **FIG. 6****.**
**FIG. 8** is a partial cross-sectional side view of a mechanical separator disposed within a collection container in an initial position for allowing fluid to pass through a through-hole in accordance with an embodiment of the present invention.
**FIG. 9a** is a partial cross-sectional side view of a mechanical separator disposed within a collection container in an intermediate position during centrifugation for allowing fluid to pass around the mechanical separator in accordance with an embodiment of the present invention.
**FIG. 9b** is a partial cross-sectional front view of the mechanical separator disposed within the collection container of **FIG. 9a** in the intermediate position during centrifugation for allowing fluid to pass around the mechanical separator in accordance with an embodiment of the present invention.
**FIG. 10a** is a partial cross-sectional side view of the mechanical separator disposed within a collection container of **FIG. 9a** in a sealed position after centrifugation in accordance with an embodiment of the present invention.
**FIG. 10b** is a partial cross-sectional front view of the mechanical separator disposed within a collection container of **FIG. 9a** in a sealed position after centrifugation in accordance with an embodiment of the present invention.

### DETAILED DESCRIPTION OF THE EMBODIMENTS

For purposes of the description hereinafter, the words "upper", "lower", "right", "left", "vertical", "horizontal", "top", "bottom", "lateral", "longitudinal", and like spatial terms, if used, shall relate to the described embodiments as oriented in the drawing figures. However, it is to be understood that many alternative variations and embodiments may be assumed except where expressly specified to the contrary. It is also to be understood that the specific devices and embodiments illustrated in the accompanying drawings and described herein are simply exemplary embodiments of the invention.

The mechanical separator of the present invention is intended for use with a collection container for providing separation of a sample into higher and lower density phase components, as will be discussed herein. For example, the present mechanical separator can be used to provide a separation of serum or plasma from whole blood through the use of differential buoyancy to cause a sealing area to contract when submerged in a specimen exposed to elevated gravitational forces through applied rotational force or centrifugation. In one embodiment, the elevated gravitational forces can be provided at a rate of at least 2,000 revolutions/minute, such as 3,400 revolutions/minute.

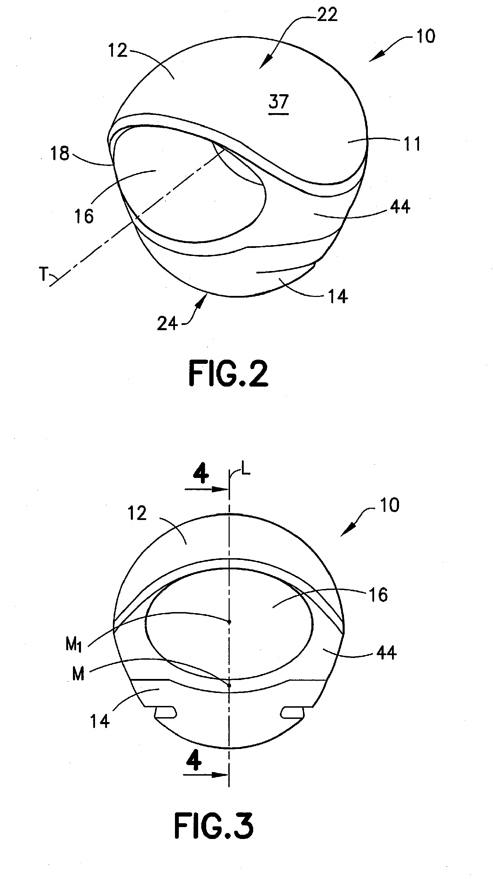

Referring to **FIGS. 2-7****,** a mechanical separator **10** of the present invention includes a separator body **11** including a first portion **12** and a second portion **14** connected to the first portion **12.** The first portion **12** has a first density and the second portion **14** has a second density, with the second density being different from the first density and, preferably, greater than the first density. Alternatively or in addition, the first portion **12** has a first buoyancy and the second portion **14** has a second buoyancy, with the second buoyancy being different from the first buoyancy, and, preferably, less than the first buoyancy.

One of the first portion **12** or the second portion **14** of the mechanical separator **10** may be extruded and/or molded of a resiliently deformable and self-sealable material, such as a thermoplastic elastomer (TPE). Alternatively, one of the first portion **12** or the second portion **14** of the mechanical separator **10** may be extruded and/or molded of a resiliently deformable material that exhibits good sealing characteristics when contact is established with a collection container, as will be discussed herein. Maintenance of the density within the specified tolerances is more easily obtained by using a standard material that does not require compounding with, for example, hollow glass micro-spheres in order to reduce the material density. The other of the first portion **12** or the second portion **14** of the mechanical separator **10** can be formed from mineral filled polypropylene.

One of the first portion **12** or the second portion **14** of the mechanical separator **10** is made from a material having a density that is less than the less-dense phase intended to be separated into two phases. For example, if it is desired to separate serum or plasma from human blood, then it is desirable that one of the first portion **12** or the second portion **14** have a density of no more than about 1.020 g/cc.

The other of the first portion **12** or the second portion **14** of the mechanical separator **10** is made from a material having a higher density than the more-dense phase intended to be separated into two phases. For example, if it is desired to separate serum or plasma from human blood, then it is desirable that the other of the first portion **12** or the second portion **14** have a density of at least 1.105 g/cc. It is anticipated herein that both the first portion **12** and the second portion **14** may be formed of various other materials with sufficient biocompatibility, density stability, additive compatibility, and neutrality to analyte interactions, adsorption, and leachability.

The mechanical separator **10** also includes a through-hole **16** defined therein, such as along a through-axis **T** of the separator body **11.** As shown in **FIGS. 2****,** **4****,** and **6****,** the through-hole **16** may extend through the entire separator body **11** and includes a first opening **18** and a second opening **20** aligned along the through-axis **T.** The through-hole **16** may bisect or substantially bisect the volumetric center of the separator body **11.** The through-hole **16** may be defined by the first portion **12** or at least a portion of the first portion **12** and at least a portion of the second portion **14.**

The first portion **12** has an exterior surface **22** that is generally arcuate in shape, such as at least partially rounded or substantially rounded. The second portion **14** also includes an exterior surface **24** that is also generally arcuate in shape, such as at least partially rounded or substantially rounded. When taken together, the exterior surface **22** of the first portion **12** and the exterior surface **24** of the second portion **14** form a generally round exterior. It is understood herein that the term "round exterior" includes configurations, in addition to a perfect sphere, that are aspects of the invention which may provide slightly nonuniform diameters taken through the mid-point. For example, different planes taken through the first portion **12** and second portion **14** which bisect the midpoint of the mechanical separator **10** may have varying diameters and still give rise to a generally rounded or ball-like mechanical separator **10.**

Due to the differential densities of the first portion **12** and the second portion **14,** the mechanical separator **10** includes a center of mass **M** that is offset from the center of volume **M1** of the separator body **11,** as shown in **FIG. 3****.** Specifically, the volume of the separator body **11** accounted for by the first portion **12** may be significantly greater than the volume of the separator body **11** accounted for by the second portion **14.** Accordingly, the center of mass **M** and center of volume **M1** of the separator body **11** may be offset from the center of the through-hole **16.**

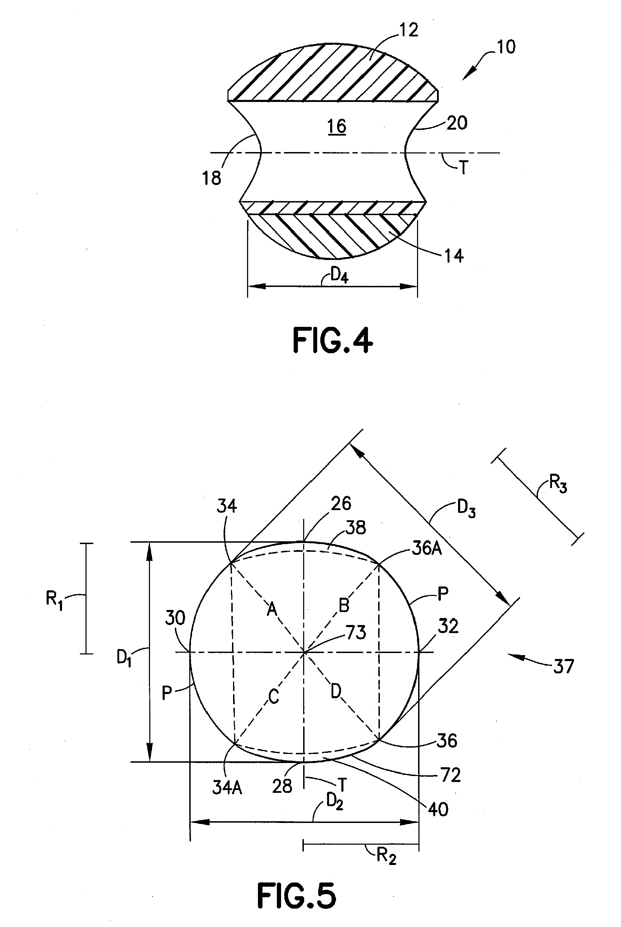

As shown in **FIG. 5****,** the top profile of the separator body **11** may be non-circular. The diameter **D₁** of the separator body **11,** specifically the first portion **12,** taken across the first portion **12** in the direction along the through-axis **T** of the through-hole **16** and extending between vertically outermost opposing tangent points **26, 28** of the perimeter **P** of the separator body **11** is less than the diameter **D₂** of the separator body **11,** specifically the first portion **12,** taken across the first portion **12** in the direction perpendicular to the through-axis **T** of the through-hole **16** and extending between laterally outermost opposing tangent points **30, 32** of the perimeter **P** of the separator body **11.** In addition, the diameter **D₃** of the separator body **11,** specifically the first portion **12,** taken across the first portion **12** at an angle substantially 45° to the through-axis **T** of the through-hole **16** and extending between diagonally outermost endpoints **34, 36** of the perimeter **P** of the separator body **11,** may be larger than the diameter of the through-hole **16,** and is greater than the diameters **D₁** and **D₂** of the separator body **11.** The diameter **D₄** of the second portion **14** taken across the second portion **14** along the through-axis **T** of the through-hole **16,** as shown in **FIG. 4****,** may be less than any of the diameters **D₁, D₂,** or **D₃** of the separator body **11.**

Referring to **FIG. 5****,** a two-dimensional projection of the top profile of the first portion **12** of the separator body **11** onto a plane may be symmetrical about an orientation plane extending between vertically outermost opposing tangent points **26, 28** of the perimeter of the separator body **11** and from a top surface **37** of the first portion **12** to a bottom surface of the second portion **14** and in the direction of the through-axis **T** of the through-hole **16.** The two-dimensional projection of the top profile of the first portion **12** of the separator body **11** onto a plane may also be symmetrical about an orientation plane extending between laterally outermost opposing tangent points **30, 32** of the perimeter **P** of the separator body **11** and from the top surface **37** of the first portion **12** to the bottom surface of the second portion **14** and perpendicular to the direction of through-axis **T** of the through-hole **16.** A two-dimensional projection of the top profile of the first portion **12** of the separator body **11** onto a plane may be asymmetrical about an orientation plane extending between diagonally outermost endpoints **34, 36** of the perimeter **P** of the separator body **11** and from the top surface **37** of the first portion **12** to the bottom surface of the second portion **14** and in a direction diagonal to at least a part of the through-axis **T** of the through-hole **16.** Accordingly, a two-dimensional projection of the top profile of the body **11** onto a plane may be asymmetric about an orientation plane extending between diagonally outermost endpoint **34A, 36A** of the perimeter **P** of the separator body **11** and from the top surface **37** of the first portion **12** to the bottom surface of the second portion **14** and in a direction diagonal to at least a part of the through-axis **T** of the through-hole **16.**

Further, the top profile of the separator body **11** defines a perimeter **P** that bounds four quadrants **A**, **B, C, D,** respectively defined by the intersection of a vertical axis extending between vertically outermost opposing tangent points **26, 28** of the perimeter **P** of the separator body **11** and a lateral axis extending between laterally outermost opposing tangent points **30, 32** of the perimeter **P** of the separator body **11.** Each quadrant **A**, **B, C, D** is substantially bisected by an orientation axis extending between diagonally outermost endpoints **34, 36 or 34A, 36A** of the perimeter **P** of the separator body **11** and bounded by the perimeter **P** of the separator body **11** as shown in **FIG. 5****.** A two-dimensional projection of the top profile of the separator body **11** onto a plane may be symmetrical about **D₁** and **D₂** but may be asymmetrical with respect to **D₃**.

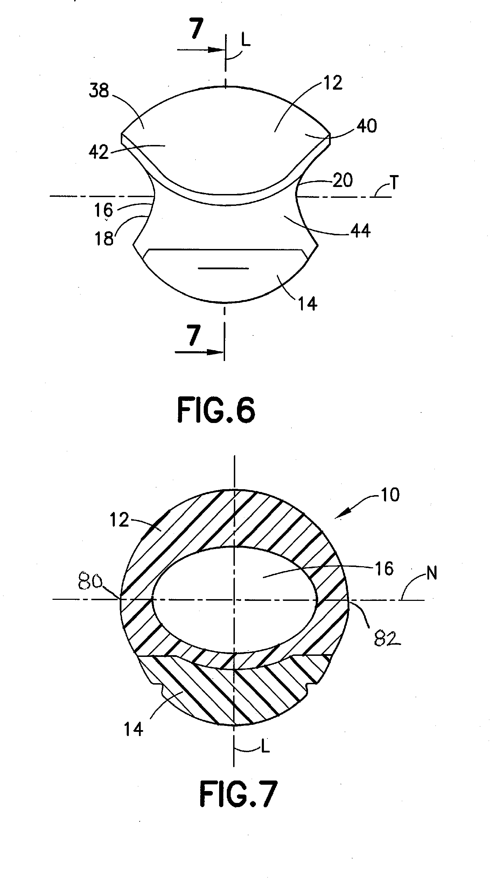

Thus, a top surface **37** of the first portion **12** includes a first extended portion **38** adjacent the first opening **18** of the through-hole **16** defined by tangent point **26,** endpoint **34,** and endpoint **36A** and a second extended portion **40** adjacent the second opening **20** of the through-hole **16** defined by tangent point **28,** endpoint **36,** and endpoint **34A,** that taken with an upper portion **42** (**FIG. 6**) of the first portion **12,** form a substantially non-circular convex top surface **37** of the first portion **12.**

As a result, the resistance to compression (compression value) or extension (extension value) of the separator body **11** to forces exerted along a longitudinal axis **L** of the separator body **11** (shown in **FIGS. 3** and **6**) extending from the top surface **37** of the first portion **12** to the bottom surface of the second portion **14** and perpendicular to the through-axis **T** of the through-hole **16** or exerted along the lateral axis **N** extending from a side **80** to a side **82** and perpendicular to the through-axis **T** of through-hole **16** is different from, and preferably less than, the resistance to compression (compression value) or extension (extension value) of the separator body **11** to forces along the through-hole axis **T** of the separator body **11** (shown in **FIG. 6****)** extending from the first opening **18** of the through-hole **16** to the second opening **20** of the through-hole **16.** The difference in compression values may be particularly noticeable when the force is a squeezing force.

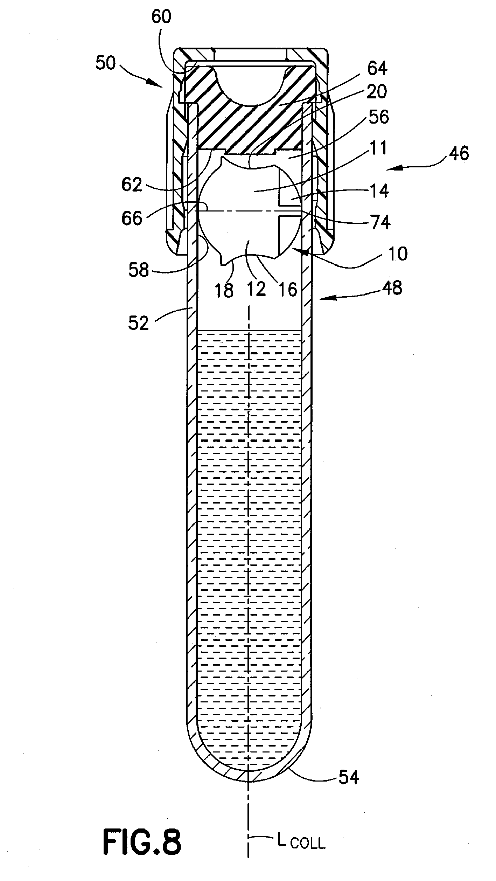

As shown in **FIGS. 8-10b****,** the mechanical separator **10** of the present invention may be provided as a portion of a separation assembly **46** for separating a fluid sample into first and second phases within a collection container **48** having a closure **50.** Specifically, the collection container **48** may be a sample collection tube, such as a proteomics, molecular diagnostics, chemistry sample tube, blood, or other bodily fluid collection tube, coagulation sample tube, hematology sample tube, and the like. The collection container **48** includes a closed bottom end **54,** an open top end **56,** and a cylindrical sidewall **52** extending therebetween. The cylindrical sidewall **52** includes an inner surface **58** with an inside diameter extending substantially uniformly from the open top end **56** to a location substantially adjacent the closed bottom end **54** along the longitudinal axis L_{COLL} of the collection container **48.**

Desirably, collection container **48** is an evacuated blood collection tube. The collection container **48** may contain additional additives as required for particular testing procedures, such as protease inhibitors, clotting agents, and the like. Such additives may be in particle or liquid form and may be sprayed onto the cylindrical sidewall **52** of the collection container **48** or located at the bottom **54** of the collection container **48.**

The collection container **48** may be made of one or more than one of the following representative materials: polypropylene, polyethylene terephthalate (PET), glass, or combinations thereof. The collection container **48** can include a single wall or multiple wall configurations. Additionally, the collection container **48** may be constructed in any practical size for obtaining an appropriate biological sample. For example, the collection container **48** may be of a size similar to conventional large volume tubes, small volume tubes, or microliter volume tubes, as is known in the art. In one particular embodiment, the collection container **48** may be a standard 13 ml evacuated blood collection tube, as is also known in the art.

The open top end **56** is structured to at least partially receive the closure **50** therein to form a liquid impermeable seal. The closure **50** includes a top end **60** and a bottom end **62** structured to be at least partially received within the collection container **48.** Portions of the closure **50** adjacent the top end **56** of the collection container **48** define a maximum outer diameter which exceeds the inside diameter of the collection container **48.** The closure **50** includes a pierceable resealable septum **64** penetrable by a needle cannula (not shown). Portions of the closure **50** extending upwardly from the bottom end **62** may taper from a minor diameter which is approximately equal to, or slightly less than, the inside diameter of the collection container **48** to a major diameter that is greater than the inside diameter of the collection container **48** at the top end **60** of the closure **50.** Thus, the bottom end **62** of the closure **50** may be urged into a portion of the collection container **48** adjacent the open top end **56** of the collection container **48.** The inherent resiliency of closure **50** can insure a sealing engagement with the inner surface **58** of the cylindrical sidewall **52** of the collection container **48.** In one embodiment, the closure **50** can be formed of a unitarily molded elastomeric material, having any suitable size and dimensions to provide sealing engagement with the collection container **48.** Optionally, the closure **50** may be at least partially surrounded by a shield, such as a Hemogard® Shield commercially available from Becton, Dickinson and Company.

As shown in **FIG. 8****,** the mechanical separator **10** of the present invention may be oriented within the collection container **48** in an initial position in which the through-hole **16** of the mechanical separator **10** is aligned with the open top end **56** of the collection container **48.** In the initial position, the through-hole **16** is adapted for allowing fluid to pass therethrough, such as from a needle cannula (not shown) which has pierced the pierceable septum **64** of the closure **50** and is provided in fluid communication with the interior of the collection container **48.** The mechanical separator **10** may also be releasably engaged with a portion of the closure **50.**

Referring to **FIG. 8****,** the initial open position of the through-hole **16** is substantially aligned with the longitudinal axis **L_{COLL}** of the collection container **48.** The mechanical separator **10** forms an interference engagement with the sidewall **52** of the collection container **48** along a first perimeter **66** as shown in **FIG. 8****.** During specimen draw into the collection container **48,** the initial separator position minimizes the accumulation of blood between the mechanical separator **10** and the closure **50.** This reduces the formation of clots and/or fibrin strands which may disrupt function of the mechanical separator **10.**

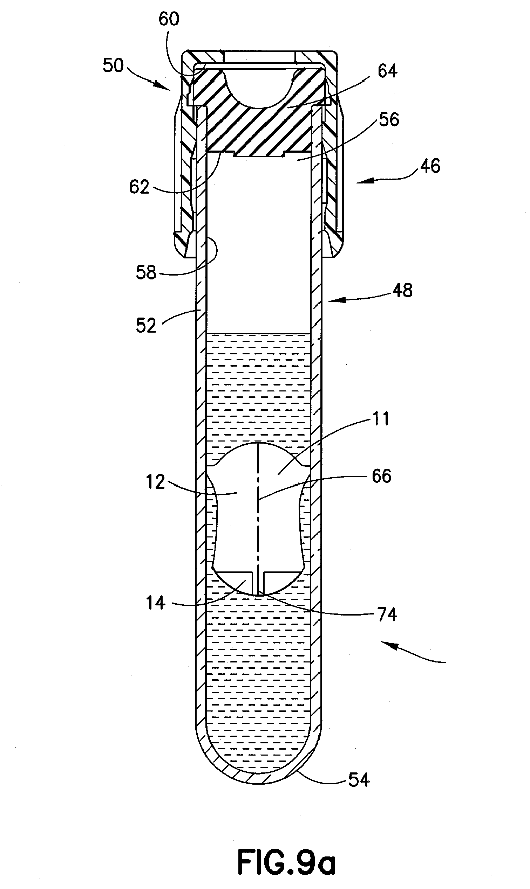

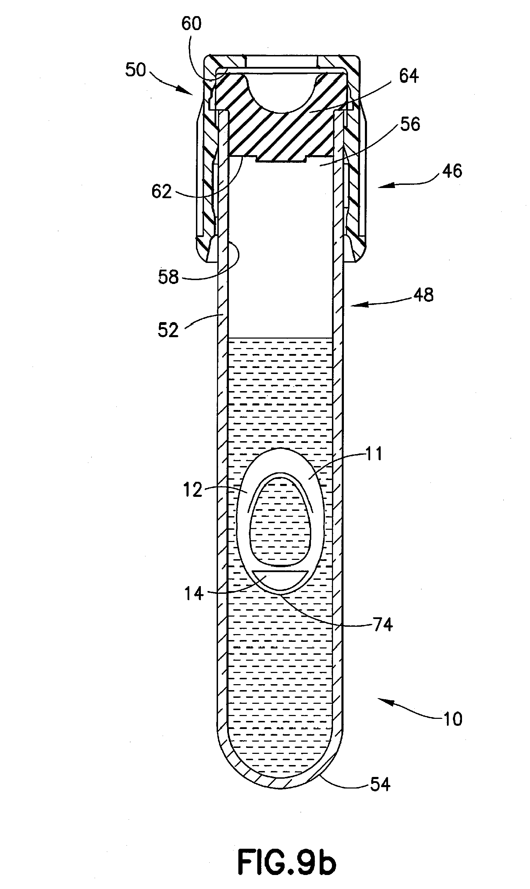

Upon application of rotational force, such as during centrifugation, and transition of the mechanical separator **10** as shown in **FIGS. 9a** and **9b****,** the mechanical separator **10** experiences a rotational moment, deforms sufficiently to disengage from engagement with the collection container **48,** and rotates approximately 90°. In the case shown in **FIGS. 8-****10b,** where the density of the first portion **12,** such as a float, is less than the density of the second portion **14,** such as a ballast, the mechanical separator **10** will be oriented with the second portion **14** facing the bottom end **54** of the collection container **48.**

Once the mechanical separator **10** contacts the fluid contained within the collection container **48,** air that occupies the through-hole **16** is progressively displaced by the fluid as the device submerges. When the mechanical separator **10** is submerged in the fluid, the difference in the buoyancy between the first portion **12** and the second portion **14** generates a differential force across the mechanical separator **10.** During centrifugation, the differential force causes the separator body **11** to elongate along the longitudinal axis **L_{COLL}** of the collection container and contract away from the sidewall **52** of the collection container **48** along the lateral axis **N,** thereby reducing the effective diameter of the separator body **11** and opening a communicative pathway for the flow of fluid, such as higher and lower density phase components, past the separator body **11.** It is noted that the first portion **12** may be adapted for deformation in a direction substantially perpendicular to the through-hole **16.**

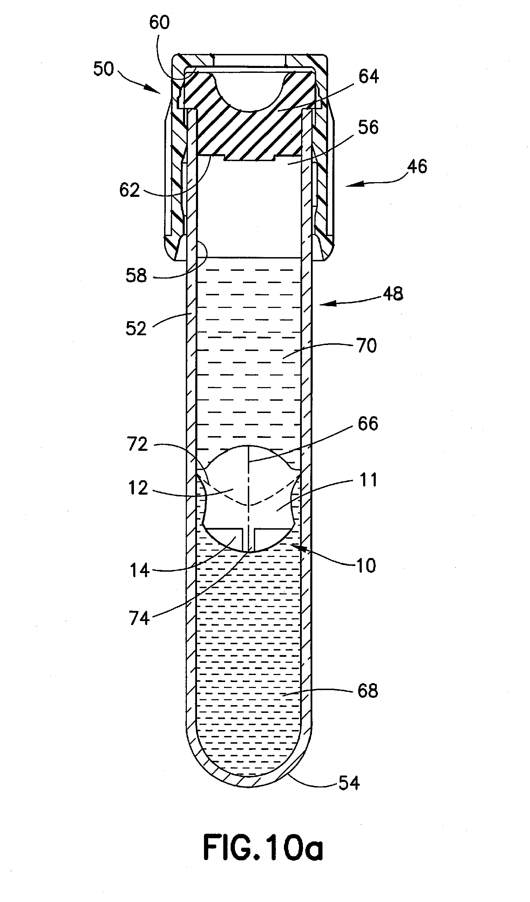

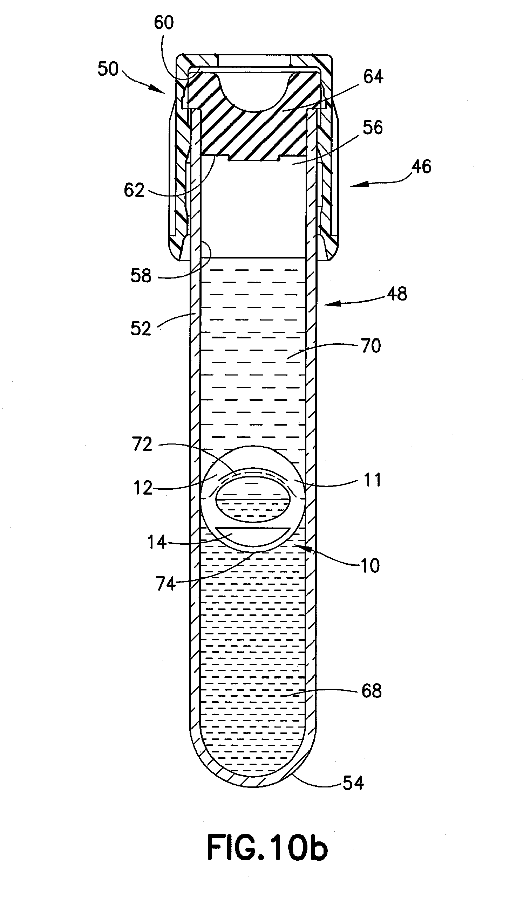

Once separation of the lower and higher density phases is complete and the application of rotational force has ceased, the mechanical separator **10** becomes oriented in a sealing position (as shown in **FIGS. 10a** **and** **10b**) between a separated higher density phase **68** and a separated lower density phase **70.** At the same time, the elongation of the separator body **11** ceases, causing the separator body **11** to return to its initial configuration, thereby forming a seal between a second outer perimeter **72** of the first portion **12** and the inner surface **58** of the sidewall **52** of the collection container **48.** The outer perimeter **72** has an outer circumference that is at least slightly larger than the interior circumference of the sidewall **52** of the collection container **48.** In addition, the smallest diameter **D₁** of the top surface **37** of the first portion **12** is at least slightly greater than the diameter of the inner surface **58** of the collection container **48.** Accordingly, the mechanical separator **10** is adapted to prevent fluid from passing between or around the separator body **11** and the collection container **48,** and also prevents fluid from passing through the through-hole **16,** effectively establishing a barrier and the second sealing perimeter **72** between higher and lower density phases within the sample.

The difference in compression and expansion values of the mechanical separator in the direction of the through-hole (separator **T** axis) versus the direction perpendicular to the through-hole (such as along the separator **L** and **N** axes) allows the separator to elongate in the longitudinal direction and contract in the lateral direction during the application of rotational force while maintaining a stabilizing separator contact with the tube inner surface **58** of sidewall **52** along the separator through-hole direction. This stabilizing contact assists in the proper movement and orientation of the separator during centrifugation. It also ensures that, upon cessation of rotational forces, the separator moves up, rather than down, to form a sealing engagement, or barrier, with the tube inner surface **58** of sidewall **52** thereby reducing the potential for contamination of the separated low density phase by the high density phase.

As can be determined from the discussions above, the separator body **11** is in a compressed, but substantially unstressed state when it forms a seal with the inner surface **58** of the sidewall **52** of the collection container **48.** The shape of the top profile of the separator body **11** provides for this compression to form a tight seal with the inner surface **58** of the sidewall **52** of the collection container **48.** The inner surface **58** of the sidewall **52** of the collection container **48** forms a first perimeter **66** shape and engagement with the separator that is substantially circular, while the separator body **11** has a top surface that defines a second perimeter **72** shape that is non-circular and provides a non-circular engagement with the inner surface **58** of sidewall **52** of collection container **48** as shown in **FIGS. 10a** and **10b**.

In order to form a tight seal between the separator body **11** and inner surface **58** of the sidewall **52** of the collection container **48,** in the substantially unstressed condition, the second perimeter **72** of the separator body **11** defines a radial distance **R₁** from a center **73** of the top surface of separator body **11** that is greater than the corresponding radius of the inner surface **58** of the sidewall **52** of the collection container **48** (**FIG. 5**). The first radial distance **R₁** may correspond to half the diameter **D₁** shown in **FIG. 5** and may be defined along a plane extending along the through-axis **T** of the through-hole **16** and perpendicular to the longitudinal axis of separator body **11** and passing through the center of the through-hole **16.** In addition, the second perimeter **72** of the separator body **11** defines at least a second radial distance **R₂** from the center **73** of the top surface of separator body **11** that is greater than the corresponding radius of the inner surface **58** of the sidewall **52** of the collection container **48.** The second radial distance **R₂** may be half the diameter **D₂** shown in **FIG. 5** and may be defined along a plane extending perpendicular to the through-hole **16** and longitudinal axis of separator body **11** and passing through the center of the through-hole **16.** The second radial distance **R₂** may be greater than the first radial distance **R₁**. Further, the second perimeter **72** of the separator body **11** may define a third radial distance **R₃** from the center **73** of the top surface of separator body **11** that is greater than the corresponding radius of the inner surface **58** of the sidewall **52** of the collection container **48.** This radial distance **R₃** may be half the diameter **D₃** shown in **FIG. 5** and may be defined along a plane representing a substantially 45° angle between the first radial distance **R₁** and the second radial distance **R₂** and perpendicular to the longitudinal axis of separator body **11.** The third radial distance **R₃** may be greater than the first radial distance **R₁** and the second radial distance **R₂.**

In the stressed condition, the second perimeter **72** of the separator body **11** defines another radial distance **R₂** from the center **73** of the top surface of separator body **11** that is slightly less than or equal to the corresponding radius of the inner surface **58** of the sidewall **52** of the collection container **48** as the separator body **11** is elongated along the longitudinal axis **L** and contracted along the lateral axis **N** during the application of rotational forces. Also, it should be noted that in the stressed and deformed condition, the second perimeter **72** of separator body **11** continues to define a radial distance **R₁** from center **73** of the top surface of separator body **11** that, unlike **R₂,** continues to be greater than the corresponding radius of the inner surface **58** of sidewall **52** of the collection container **48.**

Referring to **FIGS. 8-10b****,** the mechanical separator **10** may include an initial engagement band **74** circumferentially disposed about the separator body **11.** The initial engagement band **74** may be disposed about the separator body **11** in a direction substantially perpendicular to the through-hole **16.** The initial engagement band **74** may be continuously provided about the separator body **11,** or may optionally be provided in segments about the separator body **11.** The first portion **12** and the initial engagement band **74** may be formed from the same material, such as TPE. The initial engagement band **74** may be provided such that a first portion of the first portion **12** forms the initial engagement band **74,** and a second portion substantially bisects the second portion **14.**

As shown specifically in **FIG. 8****,** the initial engagement band **74** provides an interference engagement between the separator body **11** and the inner surface **58** of the collection container **48.** In this configuration, a first perimeter **66** about the separator body **11** is inline with the initial engagement band **74.** This first perimeter **66** assists in maintaining the separator body **11** in proper alignment with the open top end **56** of the collection container **48,** such that fluid entering the collection container **48** from a cannula (not shown) disposed through the pierceable septum **64** will pass through the first opening **18** of the through-hole **16,** through the through-hole **16,** and out the second opening **20** of the through-hole **16.**

## Claims

1. A device for separating a fluid into first and second parts within a container, comprising:
a body having a through-hole defined therethrough, the body comprising:
a first portion defining an upper surface of the body; and
a second portion defining a lower surface of the body, wherein the first portion and the second portion are interfaced,
wherein the body defines a longitudinal axis extending perpendicular to the through-hole, and wherein the body exhibits a first compression value when a force is applied to the body along the longitudinal axis,
wherein the body defines a perpendicular axis extending perpendicular to the longitudinal axis and along the through-hole, and wherein the body exhibits a second compression value when a force is applied to the body along the perpendicular axis, and
wherein the first compression value is different than the second compression value.

2. The device of claim 1, wherein the first compression value is greater than the second compression value.

3. The device of claim 1, wherein the force is exerted to the body during applied rotational force.

4. A separation assembly for enabling separation of a fluid into first and second phases, comprising:
a collection container having a first end, a second end, and a sidewall extending therebetween defining an interior; and
a separator body having a through-hole defined therethrough, the body comprising:
a first portion defining an upper surface of the body; and
a second portion defining a lower surface of the body, wherein the first portion and the second portion are interfaced,
wherein the separator body defines a longitudinal axis extending between the upper surface and the lower surface, and wherein the separator body exhibits a first compression value when a force is applied to the separator body along the longitudinal axis,
wherein the separator body defines a perpendicular axis extending perpendicular to the longitudinal axis and along the through-hole, and wherein the separator body exhibits a second compression value when a force is applied to the body along the perpendicular axis, and
wherein the first compression value is different than the second compression value.

5. The separation assembly of claim 4, wherein the first compression value is greater than the second compression value.

6. The separation assembly of claim 4, wherein the force is exerted to the body during rotational force applied to the container.

7. The separation assembly of claim 6, wherein the applied rotational force causes the separator body to elongate in a longitudinal direction and contract in a lateral direction.

8. The separation assembly of claim 7, wherein the separator body adjacent the through-hole maintains contact with the interior of the sidewall during applied rotational force.
